# EUROPEAN PATENT APPLICATION

(11) **EP 2 343 044 A1**
(43) Date of publication of application: **13.07.2011**
(21) Application number: 09817314.9
(22) Date of filing: 30.09.2009
(51) Int. Cl.: A61K 8/89, A61K 8/36

(54) **COSMETIC COMPOSITION FOR SENSITIVE SKINS**

(30) Priority: 30.09.2008 ES 200802806
(71) Applicant: Laboratorios SALVAT, S.A., 08950 Esplugues de Llobregat, Barcelona (ES)
(72) Inventor: RUIZ POL, Jaume, E-08950 Esplugues de Llobregat (Barcelona) (ES); IZQUIERDO TORRES, Francisca, E-08950 Esplugues de Llobregat (Barcelona) (ES); DE LA CRUZ TAMAYO, Daniel, E-08950 Esplugues de Llobregat (Barcelona) (ES)
(74) Representative: ABG Patentes, S.L.
(86) International application number: PCT/ES2009/000487
(87) International publication number: WO 2010/037882

(57) **Abstract**

Cosmetic composition for sensitive skins comprising hyperoxygenated fatty acids and one or more volatile silicones, together with the use thereof for the prevention of ulcers and the preservation of perilesional skin.

## Description

### Object of the Invention

The present invention relates to a cosmetic composition for sensitive skins comprising hyperoxygenated fatty acids and one or more volatile silicones, as well as to the use thereof for the prevention and healing of ulcers and the preservation of perilesional skin.

### Background of the Invention

An ulcer is understood as an open lesion of the skin in which part of the tissue has been lost and in which skin has little or no tendency to heal spontaneously, often accompanied by inflammation and sometimes by infection.

Several types of ulcers can be distinguished, among which are:
- pressure ulcers: localized areas of necrosis which appear when the soft tissue is compressed between two planes, a first plane, the bone prominences of the patient himself and the other plane, an outer surface (for example the bed), such that if the pressure does not disappear, it causes local ischemia, venous thrombosis and degenerative changes which lead to necrosis and ulceration. The prevalence of said lesions is variable, being able to range between 3 and 12% in hospitalized patients in acute care centers. These figures are higher in high-risk patients where they may reach up to 40 - 50% and may even reach 70% if elderly patients with orthopedic problems are contemplated.
- lower extremity ulcers: lower extremity ulcers of a vascular origin represent a health problem with important socioeconomic and health repercussions. Vascular ulcers are not themselves a disease, but they are always the result of a venous or arterial underlying vascular pathology. By considering their etiology, they can be classified as venous and arterial. They can be included among that which are generally known as chronic ulcers, i.e., depressed skin lesions with variable involvement, being able reach the plane of the bone from the epidermis. A common characteristic is the difficulty thereof to heal.

- diabetic foot ulcers: diabetic foot is defined as an underlying clinical etiopathogenic neuropathic alteration induced by sustained hyperglycemia, with or without ischemia, and a prior traumatic trigger produces a foot lesion and/or ulceration. In 1997, the *Sociedad Española de Medicina Familiar y Comunitaria* (Spanish Family and Community Medicine Society) calculated the approximate prevalence of diabetes mellitus in Spain at 6%. It is considered that 40-50% of diabetics develop foot ulcers throughout their life. There are two main types of diabetic foot ulcers. According to the etiology thereof, they can be neuropathic ulcers or ischemic vascular ulcers. The lower extremity neuropathic ulcer is caused by a sensory change in the lower extremity. Furthermore, it is agreed that the trigger thereof is a continuous physical-, chemical or thermal trauma that is not sufficiently perceived. Diabetic foot vascular ulcers, also known as neuroischemic ulcers, is a necrotic lesion surrounded by an erythematic halo with flat edges and therefore it lacks callous tissue which is seen in neuropathic ulcers.
- perilesional skin: perilesional skin is any skin surrounding a lesion. Its extension and appearance will depend on the location, size, type and condition of the ulcer. The concept of "perilesion" must conform to the surface which is visually circumscribed around the wound in its different particularities. For example, perilesion in the case of a heel PU is not the same as that in the case of a sacral PU. Likewise, the evaluation of the perilesional skin taking into account its condition and extension will be another indispensable element when determining which care plan must be established in the event of an ulcer. Several types of perilesional skin can be distinguished by taking into account the condition thereof: painful perilesional skin, flaking perilesional skin, intact perilesional skin, edematous perilesional skin and macerated perilesional skin. Painful perilesional skin transmits information because it may warn of situations such as: infection, unsuitable adherence of the dressing with resulting lesions, poorly controlled exudate or maceration.

### Hyperoxygenated fatty acids

Fatty acids are lipid molecules forming part of phospholipids and glycolipids and which perform different biological functions. The most important biological function is the structural function as they are part of the plasma lipid bilayer of cell membranes.

In addition, fatty acids also have a regulatory function as they are the precursors of prostaglandins, thromboxanes and leukotrienes, molecules involved in inflammatory response regulation and control, body temperature regulation and blood coagulation processes. The essential fatty acids (EFA) are those fatty acids that cannot be synthesized by the body and must be obtained through diet.

Hyperoxygenated fatty acids (HOFA) are products made up of fatty acids which have been subjected to a hyperoxygenation process. The HOFA are mild oleaginous substances which are applied on skin, helping to replace natural oils and increasing skin moisturizing and elasticity such that they reduce the incidence of ulcers and, if they are not prevented, they delay their onset time.

### Role of HOFA in pressure ulcers (PU)

HOFA have been related to the prevention of PU since the beginning. There is scientific evidence demonstrating the therapeutic efficacy of the HOFA in the prevention of PU. Different studies demonstrate the elevated effectiveness of the HOFA in preventing pressure ulcers compared to other fatty creams or discharge prevention measures.

The significant increase of blood microcirculation at the skin level, adequate skin moisturizing and elasticity they provide together with a significant reduction of the incidence of PU, maintaining skin integrity are pointed out. They further provide the skin with resistance to friction, reducing skin fragility as a result of the HOFA facilitating skin renewal.

### Role of HOFA in vascular ulcers

HOFA are especially indicated for the fragile skin of the lower extremities as a consequence of both venous and arterial vascular pathology and in the perilesional skin of lower extremity ulcers.

In clinical monitoring of patients of this type, it has been demonstrated that HOFA reduce erythema and eczema, improving skin hyperkeratosis of the extremity which is typical of such patients and they further provide relief from pruritus present in such patients.

### Role of HOFA in diabetic foot ulcers

In diabetic ulcers having an ischemic origin, HOFA show the same indications as for vascular ulcers. In addition, in diabetic ulcers of a neuropathic origin, it is advisable to use HOFA together with other general measures for the prevention thereof.

### Role of HOFA in perilesional skin

The use of HOFA in perilesional skin has recently been endorsed, being a coadjuvant treatment against the possible complications of perilesional skin. Furthermore, HOFA are optimal in preventing the onset of new lesions.

There is evidence about the benefits of using fatty acids in epidermal regeneration and in the prevention of chronic ulcers. In this sense, there is a series of products on the market containing HOFA in their formulation. One of said formulations contains hyperoxygenated glycerides of fatty acids (linolenic, linoleic, palmitic and stearic acids), phytosterols, tocopherol and perfume. Another one of the formulations contains hyperoxygenated fatty acids (mainly oleic acid), medicinal plant extracts and perfume. Another one of the formulations contains a mixture of multiple hyperoxygenated fatty acids (linoleic, oleic, linolenic, palmitic, stearic, palmitoleic, myristic, gadoleic, behenic acids), tocopherols, medicinal plant extracts and perfume.

However, said formulations have oily characteristics intrinsic to their composition which entail slow absorption once applied on the skin.

### Description of the Invention

The problem to be solved by the present invention is to provide a cosmetic composition comprising hyperoxygenated fatty acids which has good skin absorption. The solution is based on the idea that the addition of one or more volatile silicones to the composition increases the absorption thereof.

Thus, a first aspect of the present invention relates to a cosmetic composition for sensitive skins comprising hyperoxygenated fatty acids, one or more volatile silicones and one or more cosmetically acceptable excipients.

The present invention provides a cosmetic composition comprising hyperoxygenated fatty acids having excellent absorption. It also provides a cosmetic composition having an excellent ability to be extended over the skin. The cosmetic composition of the present invention also has the advantage of not leaving oily residues on the skin, which enables combining the use thereof with the application of a dressing, which is less effective with other cosmetic compositions not comprising volatile silicones that do leave oily residues since it leads to a lack of adherence on the skin due to the product itself.

Another aspect of the invention relates to the use of a cosmetic composition as described above for the prevention or treatment of ulcers.

Another aspect of the invention relates to the use of a cosmetic composition as described above for the preservation or treatment of perilesional skin.

The cosmetic composition of the present invention confers its therapeutic efficacy by significantly improving the condition of the epidermis.

On one hand, hyperoxygenated fatty acids are suitable for caring and for protecting sensitive skin in people at risk of suffering from skin ulcers.

Volatile silicone in turn allows an improved ability to extend and disperse the HOFA, as well as a rapid absorption thereof. It is colorless and does not leave a cold sensation on the skin when it evaporates. Furthermore, since it is a silicone, it protects fragile skin.

The cosmetic composition of the invention may also comprise other additional components. For example, the addition of a wetting agent allows adequate skin moisturizing, whereas the addition of an antioxidant favors skin repair.

The cosmetic composition of the invention is especially indicated in those situations requiring special care and protection of sensitive skin, such as for example the prevention and healing of ulcers, such as pressure ulcers, vascular ulcers or diabetic foot ulcers, as well as the perilesional skin due to both its reparatory effect and its protective effect.

For the purpose of the invention, the term "hyperoxygenated fatty acids (HOFA)" refers to fatty acids which have been subjected to a hyperoxygenation process controlled by oxygen saturation and which have a peroxide index comprised between 50 and 120 mEq/kg.

All the numerical values referring to the content of the different components of the cosmetic composition indicate percentages by weight, such percentages referring to the total weight of the corresponding preparation.

A particular embodiment of the invention relates to a cosmetic composition in which the hyperoxygenated fatty acids are selected from the products of the hyperoxygenation of butyric acid, caproic acid, caprylic acid, capric acid, lauric acid, lauroleic acid, myristic acid, palmitic acid, palmitoleic acid, stearic acid, oleic acid, linoleic acid, linolenic acid, arachidic acid, arachidonic acid, eicosenoic acid, behenic acid, gadoleic acid, cetoleic acid, erucic acid, lignoceric acid, cerotic acid or mixtures thereof.

Another particular embodiment relates to a cosmetic composition in which the hyperoxygenated fatty acids are obtained from fatty acids of a plant origin.

Another particular embodiment relates to a cosmetic composition in which the hyperoxygenated fatty acids comprise the products of the hyperoxygenation of a mixture of linoleic acid, oleic acid, palmitic acid and linolenic acid.

In another particular embodiment, the proportion of hyperoxygenated linoleic acid with respect to the total hyperoxygenated fatty acids can be between 25% and 90%, preferably between 30% and 80%, and more preferably between 35% and 70%.

In another particular embodiment, the proportion of hyperoxygenated oleic acid with respect to the total hyperoxygenated fatty acids can be between 5% and 60%, preferably between 10% and 50%, and more preferably between 15% and 45%.

In another particular embodiment, the proportion of hyperoxygenated palmitic acid with respect to the total hyperoxygenated fatty acids can be between 1% and 30%, preferably between 2.5% and 25%, and more preferably between 5% and 20%.

In another particular embodiment, the proportion of hyperoxygenated linolenic acid with respect to the total hyperoxygenated fatty acids can be between 0.1% and 5%, preferably between 0.2% and 3%, and more preferably between 0.4% and 2.5%.

In another particular embodiment, the hyperoxygenated fatty acids additionally comprise products of the hyperoxygenation of stearic acid, arachidic acid, eicosenoic acid or behenic acid, or any of the mixtures thereof.

In another particular embodiment, the proportion of hyperoxygenated stearic acid with respect to the total hyperoxygenated fatty acids is less than or equal to 15%, preferably less than or equal to 10%, and more preferably less than or equal to 5%.

In another particular embodiment, the proportion of hyperoxygenated arachidic, eicosenoic or behenic acids with respect to the total hyperoxygenated fatty acids is less than or equal to 10%, preferably less than or equal to 5%, and more preferably less than or equal to 1%.

The term "volatile silicone" refers to a silicone having a heat of vaporization lower than the heat of vaporization of water, which is 2257 kJ/kg at 25 °C.

In another particular embodiment, the volatile silicone is selected from the group consisting of low viscosity linear siloxanes, branched siloxanes, cyclic siloxanes and silicone copolymers.

In another particular embodiment, the volatile silicone is a cyclic siloxane.

In another particular embodiment, the volatile silicone is cyclomethicone.

In another particular embodiment, the cyclomethicone is cyclopentasiloxane or a mixture of cyclopentasiloxane and cyclotetrasiloxane.

The cosmetic composition of the present invention comprises one or more cosmetically acceptable excipients selected from the group consisting of emulsifiers, solubilizing agents, dispersants, wetting agents, co-emulsifiers, emollients, surfactants, thickening agents, viscosity increasing agents, lipid components for increasing the consistency of the emulsion, preservatives, pH adjusting agents, flavoring agents and perfumes, or any of the mixtures thereof.

In a particular embodiment, the cosmetically acceptable excipients can be liquids or solids and of an aqueous, lipid or organic nature.

Another particular embodiment relates to a cosmetic composition which is formulated for topical or dermatological application.

Another particular embodiment relates to a cosmetic composition which is formulated in the form of an oil, emulsion, ointment, cream, microemulsion, solution, gel, foam, paste, lotion, poultice or any other form for topical or dermatological application that can be conceived by a person skilled in the art and can be formulated in single or multiple-dose form.

Another particular embodiment relates to a cosmetic composition which is formulated in the form of an oil.

In another particular embodiment, the hyperoxygenated fatty acid content is comprised between 50% and 99% by weight, preferably between 65% and 95% by weight, and more preferably between 80% and 90% by weight, such percentage referring to the total weight of the preparations.

In another particular embodiment, the volatile silicone content is comprised between 0.5% and 15% by weight, preferably between 1% and 10% by weight, and more preferably between 2.5% and 7.5% by weight, such percentage referring to the total weight of the preparations.

In another particular embodiment, the cosmetic composition comprises an emollient or mixture of emollients selected from the group consisting of isostearic acid, isopropyl myristate, isopropyl isostearate, isopropyl palmitate, diisopropyl dilinoleate, glyceryl tricaprate/tricaprylate, glyceryl tricaprylate/tricaprate, glyceryl trioctanoate, medium chain triglycerides, caprylic/capric triglycerides, squalane, squalene, jojoba oil, mineral oil and petroleum jelly, or any of the mixtures thereof.

In another particular embodiment, the emollient or mixture of emollients content is comprised between 1% and 30% by weight, preferably between 2.5% and 25%, and more preferably between 5% and 15% by weight, such percentage referring to the total weight of the preparations.

In another particular embodiment, the cosmetic composition is formulated in the form of an emulsion.

In another particular embodiment, the hyperoxygenated fatty acid content is comprised between 10% and 60% by weight, preferably between 25% and 50% by weight, and more preferably between 35% and 45% by weight, such percentage referring to the total weight of the preparations.

In another particular embodiment, the volatile silicone content is comprised between 0.1% and 10% by weight, preferably between 0.5% and 8% by weight, and more preferably between 1% and 5% by weight, such percentage referring to the total weight of the preparations.

In another particular embodiment, the cosmetic composition additionally comprises water.

In another particular embodiment, the water content is comprised between 40% and 90% by weight, preferably between 50% and 75% by weight, and more preferably between 55% and 65% by weight, such percentage referring to the total weight of the preparations.

In another particular embodiment, the cosmetic composition additionally comprises a surfactant.

In another particular embodiment, the surfactant content is comprised between 0.05% and 10% by weight, preferably between 0.5% and 8% by weight, and more preferably between 1% and 5% by weight, such percentage referring to the total weight of the preparations.

In another particular embodiment, the surfactant is a non-ionic surfactant selected from the group consisting of alkanolamides and derivatives thereof, ethoxylated amides, amine oxides, esters (such as ethoxylated carboxylic acids, ethoxylated glycerides, glycol esters and derivatives, monoglycerides, polyglyceryl esters, polyalcohol esters, sorbitan esters, sorbitol esters, phosphoric acid triesters), alcohols and derivatives thereof and ethers (such as ethoxylated alcohols, ethoxylated lanolin derivatives, ethoxylated polysiloxanes, propoxylated PEG ethers, polyalcohol ethers), or mixtures thereof.

In another particular embodiment, the cosmetic composition additionally comprises a lipid component for increasing the consistency of the emulsion selected from the group consisting of cetyl alcohol and derivatives thereof, lauryl alcohol, myristyl alcohol, cetearyl alcohol, stearyl alcohol, behenyl alcohol, glyceryl stearate and cocoglycerides and derivatives thereof, or mixtures thereof.

In another particular embodiment, the lipid component content is comprised between 1% and 30% by weight, preferably between 2% and 20% by weight, and more preferably between 5% and 15% by weight, such percentage referring to the total weight of the preparations.

In another particular embodiment, the cosmetic composition additionally comprises an antioxidant in a percentage comprised between 0.1% and 2% by weight, such percentage referring to the total weight of the preparations. Said antioxidant is selected from the group consisting of tocopherols and derivatives thereof (such as tocopherol acetate), vitamin A and derivatives thereof (such as vitamin A palmitate), folic acid and derivatives thereof, amino acids (such as glycine, histidine, tyrosine, tryptophan) and derivatives thereof, imidazoles (such as urocanic acid) and derivatives thereof, carotenoids, carolines and derivatives thereof, lipoic acid and derivatives thereof, butylhydroxytoluene, butylhydroxyanisole, uric acid and derivatives thereof, selenium and derivatives thereof, zinc and derivatives thereof, citric acid, EDTA, EGTA, lactic acid and malic acid.

### Examples

The following examples aim to illustrate the present invention without limiting it. The numerical values of the examples indicate percentages by weight, such percentages referring to the total weight of the corresponding preparation. Some practical cases of its use are also presented by way of example.

In the following examples, the term "HOFA" refers to an oil which comprises a mixture of hyperoxygenated fatty acids having a composition according to the following table:

| | |
|---|---|
| Fatty acids < C 16 | ≤ 0.6 % |
| Palmitic acid C 16 | 8.6 - 16.5 % |
| Stearic acid C 18 | ≤ 3.3 % |
| Oleic acid C 18:1 | 20.0 - 42.2 % |
| Linoleic acid C 18:2 | 39.4 - 65.6 % |
| Linolenic acid C 18:3 | 0.5 - 1.5 % |
| Arachidic acid C 20 | ≤ 0.8 % |
| Eicosenoic acid C 20:1 | ≤ 0.5 % |
| Behenic acid C 22 | ≤ 0.5 % |
| Other acids | ≤ 0.5 % |

The fatty acid composition of the oil is characteristic of each batch and can vary from batch to batch.

### Example 1: Cosmetic composition in the form of an oil 1

| COMPOSITION | CONCENTRATION (% w/w) |
|---|---|
| HOFA | 85.5 |
| Cyclomethicone | 6.0 |
| Caprylic/capric triglycerides | 7.5 |
| Other excipients | 1.0 |

### Example 2: Cosmetic composition in the form of an oil 2

| COMPOSITION | CONCENTRATION (% w/w) |
|---|---|
| HOFA | 81.5 |
| Cyclomethicone | 7.5 |
| Caprylic/capric triglycerides | 5.0 |
| Squalane | 5.0 |
| Other excipients | 1.0 |

### Example 3: Cosmetic composition in the form of an oil 3

| COMPOSITION | CONCENTRATION (% w/w) |
|---|---|
| HOFA | 83.5 |
| Cyclomethicone | 5.0 |
| Caprylic/capric triglycerides | 10.0 |
| Other excipients | 1.5 |

### Example 4: Cosmetic composition in the form of an oil 4

| COMPOSITION | CONCENTRATION (% w/w) |
|---|---|
| HOFA | 87.5 |
| Cyclomethicone | 2.5 |
| Caprylic/capric triglycerides | 7.5 |
| Other excipients | 2.5 |

### Example 5: Cosmetic composition in the form of an emulsion 1

| COMPOSITION | CONCENTRATION (% w/w) |
|---|---|
| HOFA | 38.0 |
| Cyclomethicone | 2.0 |
| Cetearyl alcohol | 6.0 |
| Propylene glycol | 3.0 |
| Tocopherol acetate | 0.4 |
| Perfume | 0.6 |
| Other excipients | 5.0 |
| Sodium hydroxide | 0.1 |
| Water | 44.9 |

### Example 6: Cosmetic composition in the form of an emulsion 2

| COMPOSITION | CONCENTRATION (% w/w) |
|---|---|
| HOFA | 30.0 |
| Cyclomethicone | 3.0 |
| Glyceryl stearate | 8.0 |
| Propylene glycol | 4.0 |
| Tocopherol acetate | 0.5 |
| Perfume | 0.8 |
| Other excipients | 10.0 |
| Sodium hydroxide | 0.1 |
| Water | 43.6 |

### Example 7: Cosmetic composition in the form of an emulsion 3

| COMPOSITION | CONCENTRATION (% w/w) |
|---|---|
| HOFA | 25.0 |
| Cyclomethicone | 5.0 |
| Cetyl alcohol | 6.0 |
| Propylene glycol | 5.0 |
| Tocopherol acetate | 0.3 |
| Perfume | 0.9 |
| Other excipients | 15.0 |
| Sodium hydroxide | 0.1 |
| Water | 42.7 |

### Example 8: Wounds after bimalleolar left ankle fracture surgery

A 61 year old patient having two surgical wounds after a bimalleolar left ankle fracture with a loss of skin integrity was treated.

Said patient had a 10 cm scar in the inner malleolus with a 4 x 0.5 cm stage II pressure ulcer, without signs of infection, with little exudate and granulation tissue which appeared after removing the eschar, as well as a scar in the outer malleolus 15 cm long and 0.3 - 0.5 cm wide. Both wounds had perilesional skin with violaceous erythema and dry eczema with flaking. The presence of edema (due to the immobility caused by a boot) and dry extremity with flaking which caused intense pruritus to the patient was observed in the entire lower left extremity.

The lesion was treated by means of cleaning with physiological serum, antiseptic gel and gauze dressing. The cosmetic composition was applied daily on the entire extremity. The lesion of the inner malleolus healed 7 days after starting the treatment, whereas the lesion of the outer malleolus healed in 24 days. The perilesional skin in turn visibly improved in only two days after applying the cosmetic composition. The elimination of the pruritus further increased the patient's comfort, such that skin of the extremity damaged by immobility after the fracture was repaired, achieving a normal appearance and color and providing a greater moisturizing to achieve a faster healing of the lesion.

### Example 9: Superficial 2nd degree burn on right arm

A 65 year old patient who came to the nursing consultation to treat a burn wound on the right arm with a loss of skin integrity was treated.

Said patient had a 15.5 x 6 cm burn with a progress of 13 days located on the outer part of the right arm. He did not have any phlyctena upon arrival as they had been removed in the previous treatments, and he showed no signs of infection but significant pain and moderate exudate. The granulation tissue had an extension of 3 x 2.7 cm, whereas epidermis had already been formed in the rest of the burn.

The lesion was treated by means of cleaning the perilesional skin every 24 hours with physiological serum, silver sulfadiazine, non-adhesive Skinfoam secured with a bandage and the cosmetic composition.

The resolution of the burn was observed in 13 days, and it was in accordance with the characteristics thereof, as well as the dyschromia of the lesioned skin which was gradually normalized with the passing of the days, indicating that the application of the cosmetic composition is a good therapeutic option to prevent sequelae in burns since the skin was flexible, and the pruritus which normally accompanies such scars had been eliminated.

### Example 10: Pressure ulcer (PU)

A 76 year old patient who had ulcers in the coccyx and perianal area due to the effects of a diaper and pressure, with a loss of skin integrity, was treated.

Said patient had an ulcer with a progress of four days of approximately 15 x 7 cm which was in fact not a single ulcer but an aggregation of several Stage II PU with very erythematic perilesional skin, without signs of infection and with little exudate.

The lesion was treated by means of cleaning with physiological serum, the cosmetic composition and as a secondary dressing, adhesive foam which was correctly maintained between the changes. The care taker treated the wound every 12 hours and the nurse controlled the progress of the area every 3 days.

The healing of the ulcer was observed after 10 days due to adequate skin moisturizing by the cosmetic composition and the possibility of being able to place a foam dressing such as a Skinfoam with complete adherence.

### Example 11: Vascular venous ulcer

A 69 year old patient who came to the nursing consultation to treat a vascular varicose ulcer in the lower left extremity with a loss of skin integrity due to the intermittent presence of a venous vascular ulcer was treated.

Said patient had a 3 x 1.5 cm ulcer covered with a scab, with an involved perilesional area 10 cm in diameter with eczema and erythema.

The lesion was treated by means of cleaning the ulcer area as well as the entire extremity with physiological serum and the cosmetic composition.

The healing of the ulcer, good perilesional skin condition as well as a significant reduction of erythema present on the first day and an extremity with a suitable level of moisturizing was observed after 5 weeks, which indicates that the cosmetic composition is suitable for vascular ulcers, reducing erythema, eczema and pruritus at the same time.

### Example 12: Prevention of vascular venous ulcer recurrence

A 73 year old patient in whom treatment with the cosmetic composition as prevention of vascular venous ulcer recurrence had been started was treated.

Said patient had very fragile skin, violaceous erythema or redness and a lot of flaking on the lower two-thirds of both extremities.

The lesion was treated by means of applying the cosmetic composition every 12 hours and a weekly evaluation in the nursing consultancy.

The improvement of the appearance, the disappearance of flaking and the reduction of pruritus was observed, therefore the application of the cosmetic composition is a good therapeutic option to prevent the onset of vascular ulcers in patients with this pathology, even in those who have a very fragile skin due to the deterioration of skin microcirculation.

## Claims

1. A cosmetic composition for sensitive skins, **characterized in that** it comprises hyperoxygenated fatty acids, one or more volatile silicones and one or more cosmetically acceptable excipients.

2. The cosmetic composition according to claim 1, wherein the hyperoxygenated fatty acids are obtained from fatty acids of a plant origin.

3. The cosmetic composition according to claim 1, wherein the hyperoxygenated fatty acids are selected from the products of the hyperoxygenation of butyric acid, caproic acid, caprylic acid, capric acid, lauric acid, lauroleic acid, myristic acid, palmitic acid, palmitoleic acid, stearic acid, oleic acid, linoleic acid, linolenic acid, arachidic acid, arachidonic acid, eicosenoic acid, behenic acid, gadoleic acid, cetoleic acid, erucic acid, lignoceric acid, cerotic acid or mixtures thereof.

4. The cosmetic composition according to any of claims 1 to 3, wherein the hyperoxygenated fatty acids comprise the products of the hyperoxygenation of a mixture of linoleic acid, oleic acid, palmitic acid and linolenic acid.

5. The cosmetic composition according to claim 4, wherein the proportion of hyperoxygenated linoleic acid with respect to the total hyperoxygenated fatty acids is between 25% and 90%.

6. The cosmetic composition according to claim 5, wherein the proportion of hyperoxygenated linoleic acid with respect to the total hyperoxygenated fatty acids is between 30% and 80%.

7. The cosmetic composition according to claim 6, wherein the proportion of hyperoxygenated linoleic acid with respect to the total hyperoxygenated fatty acids is between 35% and 70%.

8. The cosmetic composition according to any of claims 4 to 6, wherein the proportion of hyperoxygenated oleic acid with respect to the total hyperoxygenated fatty acids is between 5% and 60%.

9. The cosmetic composition according to claim 8, wherein the proportion of hyperoxygenated oleic acid with respect to the total hyperoxygenated fatty acids is between 10% and 50%.

10. The cosmetic composition according to claim 9, wherein the proportion of hyperoxygenated oleic acid with respect to the total hyperoxygenated fatty acids is between 15% and 45%.

11. The cosmetic composition according to any of claims 4 to 10, wherein the proportion of hyperoxygenated palmitic acid with respect to the total hyperoxygenated fatty acids is between 1% and 30%.

12. The cosmetic composition according to claim 11, wherein the proportion of hyperoxygenated palmitic acid with respect to the total hyperoxygenated fatty acids is between 2.5% and 25%.

13. The cosmetic composition according to claim 12, wherein the proportion of hyperoxygenated palmitic acid with respect to the total hyperoxygenated fatty acids is between 5% and 20%.

14. The cosmetic composition according to any of claims 4 to 13, wherein the proportion of hyperoxygenated linolenic acid with respect to the total hyperoxygenated fatty acids is between 0.1% and 5%.

15. The cosmetic composition according to claim 14, wherein the proportion of hyperoxygenated linolenic acid with respect to the total hyperoxygenated fatty acids is between 0.2% and 3%.

16. The cosmetic composition according to claim 15, wherein the proportion of hyperoxygenated linolenic acid with respect to the total hyperoxygenated fatty acids is between 0.4% and 2.5%.

17. The cosmetic composition according to any of claims 4 to 16, wherein the hyperoxygenated fatty acids additionally comprise products of the hyperoxygenation of stearic acid, arachidic acid, eicosenoic acid or behenic acid, or any of the mixtures thereof.

18. The cosmetic composition according to claim 17, wherein the proportion of hyperoxygenated stearic acid with respect to the total hyperoxygenated fatty acids is less than or equal to 15%.

19. The cosmetic composition according to claim 18, wherein the proportion of hyperoxygenated stearic acid with respect to the total hyperoxygenated fatty acids is less than or equal to 10%.

20. The cosmetic composition according to claim 19, wherein the proportion of hyperoxygenated stearic acid with respect to the total hyperoxygenated fatty acids is less than or equal to 5%.

21. The cosmetic composition according to any of claims 17 to 20, wherein the proportion of hyperoxygenated arachidic, eicosenoic or behenic acids with respect to the total hyperoxygenated fatty acids is less than or equal to 10%.

22. The cosmetic composition according to claim 21, wherein the proportion of hyperoxygenated arachidic, eicosenoic or behenic acids with respect to the total hyperoxygenated fatty acids is less than or equal to 5%.

23. The cosmetic composition according to claim 22, wherein the proportion of hyperoxygenated arachidic, eicosenoic or behenic acids with respect to the total hyperoxygenated fatty acids is less than or equal to 1%.

24. The cosmetic composition according to any of claims 1 to 23, wherein the volatile silicone is selected from the group consisting of low viscosity linear siloxanes, branched siloxanes, cyclic siloxanes and silicone copolymers.

25. The cosmetic composition according to claim 24, wherein the volatile silicone is a cyclic siloxane.

26. The cosmetic composition according to claim 24, wherein the volatile silicone is cyclomethicone.

27. The cosmetic composition according to claim 26, wherein the cyclomethicone is cyclopentasiloxane or a mixture of cyclopentasiloxane and cyclotetrasiloxane.

28. The cosmetic composition according to any of claims 1 to 27, **characterized in that** the cosmetically acceptable excipients are selected from the group consisting of emulsifiers, solubilizing agents, dispersants, wetting agents, co-emulsifiers, emollients, surfactants, thickening agents, viscosity increasing agents, lipid components for increasing the consistency of the emulsion, preservatives, pH adjusting agents, flavoring agents and perfumes, or any of the mixtures thereof.

29. The cosmetic composition according to claim 28, **characterized in that** the cosmetically acceptable excipients are liquids or solids and of an aqueous, lipid, organic or inorganic nature.

30. The cosmetic composition according to any of the preceding claims, **characterized in that** it is formulated for topical or dermatological application.

31. The cosmetic composition according to claim 30, **characterized in that** it is formulated in the form of an oil, emulsion, ointment, cream, microemulsion, solution, gel, foam, paste, lotion, poultice or any other form for topical or dermatological application.

32. The cosmetic composition according to any of the preceding claims, **characterized in that** it is formulated in single or multiple dose form.

33. The cosmetic composition according to claim 31, **characterized in that** it is formulated in the form of an oil.

34. The cosmetic composition according to claim 33, **characterized in that** the hyperoxygenated fatty acid content is comprised between 50% and 99% by weight, such percentage referring to the total weight of the preparations.

35. The cosmetic composition according to claim 34, **characterized in that** the hyperoxygenated fatty acid content is comprised between 65% and 95% by weight, such percentage referring to the total weight of the preparations.

36. The cosmetic composition according to claim 35, **characterized in that** the hyperoxygenated fatty acid content is comprised between 80% and 90% by weight, such percentage referring to the total weight of the preparations.

37. The cosmetic composition according to any of claims 33 to 36, **characterized in that** the volatile silicone content is comprised between 0.5% and 15% by weight, such percentage referring to the total weight of the preparations.

38. The cosmetic composition according to claim 37, **characterized in that** the volatile silicone content is comprised between 1% and 10% by weight, such percentage referring to the total weight of the preparations.

39. The cosmetic composition according to claim 38, **characterized in that** the volatile silicone content is comprised between 2.5% and 7.5% by weight, such percentage referring to the total weight of the preparations.

40. The cosmetic composition according to any of claims 33 to 39, **characterized in that** it comprises an emollient or a mixture of emollients.

41. The cosmetic composition according to claim 40, wherein the emollient is selected from the group consisting of isostearic acid, isopropyl myristate, isopropyl isostearate, isopropyl palmitate, diisopropyl dilinoleate, glyceryl tricaprate/tricaprylate, glyceryl tricaprylate/tricaprate, glyceryl trioctanoate, medium chain triglycerides, caprylic/capric triglycerides, squalane, squalene, jojoba oil, mineral oil and petroleum jelly, or any of the mixtures thereof.

42. The cosmetic composition according to any of claims 40 to 41, **characterized in that** the emollient or mixture of emollients content is comprised between 1% and 30% by weight, such percentage referring to the total weight of the preparations.

43. The cosmetic composition according to claim 42, **characterized in that** the emollient or mixture of emollients content is comprised between 2.5% and 25% by weight, such percentage referring to the total weight of the preparations.

44. The cosmetic composition according to claim 43, **characterized in that** the emollient or mixture of emollients content is comprised between 5% and 15% by weight, such percentage referring to the total weight of the preparations.

45. The cosmetic composition according to claim 31, **characterized in that** it is formulated in the form of an emulsion.

46. The cosmetic composition according to claim 45, **characterized in that** the hyperoxygenated fatty acid content is comprised between 10% and 60% by weight, such percentage referring to the total weight of the preparations.

47. The cosmetic composition according to claim 46, **characterized in that** the hyperoxygenated fatty acid content is comprised between 25% and 50% by weight, such percentage referring to the total weight of the preparations.

48. The cosmetic composition according to claim 47, **characterized in that** the hyperoxygenated fatty acid content is comprised between 35% and 45% by weight, such percentage referring to the total weight of the preparations.

49. The cosmetic composition according to any of claims 45 to 48, **characterized in that** the volatile silicone content is comprised between 0.1% and 10% by weight, such percentage referring to the total weight of the preparations.

50. The cosmetic composition according to claim 49, **characterized in that** the volatile silicone content is comprised between 0.5% and 8% by weight, such percentage referring to the total weight of the preparations.

51. The cosmetic composition according to claim 50, **characterized in that** the volatile silicone content is comprised between 1% and 5% by weight, such percentage referring to the total weight of the preparations.

52. The cosmetic composition according to any of claims 45 to 51, **characterized in that** it comprises water.

53. The cosmetic composition according to claim 52, **characterized in that** the water content is comprised between 40% and 90% by weight, such percentage referring to the total weight of the preparations.

54. The cosmetic composition according to claim 53, **characterized in that** the water content is comprised between 50% and 75% by weight, such percentage referring to the total weight of the preparations.

55. The cosmetic composition according to claim 54, **characterized in that** the water content is comprised between 55% and 65% by weight, such percentage referring to the total weight of the preparations.

56. The cosmetic composition according to any of claims 45 to 55, **characterized in that** it additionally comprises a surfactant.

57. The cosmetic composition according to claim 56, **characterized in that** the surfactant content is comprised between 0.05% and 10% by weight, such percentage referring to the total weight of the preparations.

58. The cosmetic composition according to claim 57, **characterized in that** the surfactant content is comprised between 0.5% and 8% by weight, such percentage referring to the total weight of the preparations.

59. The cosmetic composition according to claim 58, **characterized in that** the surfactant content is comprised between 1% and 5% by weight, such percentage referring to the total weight of the preparations.

60. The cosmetic composition according to any of claims 56 to 59, **characterized in that** the surfactant is a non-ionic surfactant.

61. The cosmetic composition according to claim 60, wherein the non-ionic surfactant is selected from the group consisting of alkanolamides and derivatives thereof; ethoxylated amides; amine oxides; esters, such as ethoxylated carboxylic acids, ethoxylated glycerides, glycol esters and derivatives, monoglycerides, polyglyceryl esters, polyalcohol esters, sorbitan esters, sorbitol esters, phosphoric acid triesters; alcohols and derivatives thereof; and ethers, such as ethoxylated alcohols, ethoxylated lanolin derivatives, ethoxylated polysiloxanes, propoxylated PEG ethers, polyalcohol ethers; or mixtures thereof.

62. The cosmetic composition according to any of claims 45 to 61, **characterized in that** it additionally comprises a lipid component for increasing the consistency of the emulsion.

63. The cosmetic composition according to claim 62, **characterized in that** the lipid component content is comprised between 1% and 30% by weight, such percentage referring to the total weight of the preparations.

64. The cosmetic composition according to claim 63, **characterized in that** the lipid component content is comprised between 2% and 20% by weight, such percentage referring to the total weight of the preparations.

65. The cosmetic composition according to claim 64, **characterized in that** the lipid component content is comprised between 5% and 15% by weight, such percentage referring to the total weight of the preparations.

66. The cosmetic composition according to any of claims 62 to 65, wherein the lipid component for increasing the consistency of the emulsion is selected from the group consisting of cetyl alcohol and derivatives thereof, lauryl alcohol, myristyl alcohol, cetearyl alcohol, stearyl alcohol, behenyl alcohol, glyceryl stearate and cocoglycerides and derivatives thereof, or mixtures thereof.

67. The cosmetic composition according to any of claims 45 to 66, **characterized in that** it additionally comprises an antioxidant in a percentage comprised between 0.1% and 2% by weight, such percentage referring to the total weight of the preparations.

68. The cosmetic composition according to claim 67, wherein the antioxidant is selected from the group consisting of tocopherols and derivatives thereof, such as tocopherol acetate; vitamin A and derivatives thereof, such as vitamin A palmitate; folic acid and derivatives thereof; amino acids such as glycine, histidine, tyrosine, tryptophan, and derivatives thereof; imidazoles such as urocanic acid, and derivatives thereof; carotenoids; carolines and derivatives thereof; lipoic acid and derivatives thereof; butylhydroxytoluene; butylhydroxyanisole; uric acid and derivatives thereof; selenium and derivatives thereof; zinc and derivatives thereof; citric acid; EDTA; EGTA; lactic acid and malic acid.

69. Use of a cosmetic composition according to any of the preceding claims for the prevention or treatment of ulcers.

70. Use of a cosmetic composition according to any of the preceding claims for the preservation or treatment of perilesional skin.
